# EUROPÄISCHE PATENTSCHRIFT

(11) **EP 3 122 712 B1**
(45) Veröffentlichungstag und Bekanntmachung des Hinweises auf die Patenterteilung: **13.02.2019**
(21) Anmeldenummer: 15741826.0
(22) Anmeldetag: 25.03.2015
(51) Int. Cl.: C07C 2/78, C01B 3/36, B01J 7/00

(54) **VORRICHTUNG UND VERFAHREN ZUM HERSTELLEN VON ACETYLEN UND SYNTHESEGAS**
DEVICE AND METHOD FOR PRODUCING ACETYLENE AND SYNTHESIS GAS
DISPOSITIF ET PROCÉDÉ DE PRODUCTION D'ACÉTYLÈNE ET DE GAZ DE SYNTHÈSE

(30) Priorität: 26.03.2014 EP 14161692
(43) Veröffentlichungstag der Anmeldung: 01.02.2017
(73) Patentinhaber: BASF SE, 67056 Ludwigshafen am Rhein (DE)
(72) Erfinder: VICARI, Maximilian, 67117 Limburgerhof (DE); BRITZIUS, Susanne, 68239 Mannheim (DE); KARA-HIPPEN, Lilian, 67227 Frankenthal (DE); RUSS, Michael, 67354 Römerberg (DE); KERN, Matthias, 67146 Deidesheim (DE); WEICHERT, Christian, 67146 Deidesheim (DE)
(86) Internationale Anmeldenummer: PCT/EP2015/056377
(87) Internationale Veröffentlichungsnummer: WO 2015/144754

(56) Entgegenhaltungen:
- WO-A1-2013/186291
- WO-A1-2014/037311

## Beschreibung

Die vorliegende Erfindung betrifft eine verbesserte Vorrichtung und ein verbessertes Verfahren zum Herstellen von Acetylen und Synthesegas durch partielle Oxidation von Kohlenwasserstoffen in einem Reaktor, bei welchen man dem Reaktor einen den Kohlenwasserstoff enthaltenden Strom sowie einen den Sauerstoff enthaltenden Strom zuleitet.

Die WO 2014/037311 A1 und WO 2013/186291 A1 offenbaren jeweils ein Verfahren zum Herstellen von Acetylen und Synthesegas.

Hochtemperaturreaktionen zur partiellen Oxidation von Kohlenwasserstoffen werden üblicherweise in einem Reaktorsystem aus Mischeinheit, Brennerblock, Feuerraum und Quencheinrichtung durchgeführt. Als Beispiel für eine solche partielle Oxidation im Hochtemperaturbereich ist die Herstellung von Acetylen und Synthesegas durch partielle Oxidation von Kohlenwasserstoffen zu nennen. Diese wird beispielsweise in der DE 875198, DE 1051845, DE 1057094 und DE 4422815 beschrieben.

Hierin werden die für das BASF-Sachsse-Bartholome-Acetylenverfahren üblicherweise eingesetzten Mischer/Brennerblock/Feuerraum/Quench-Kombinationen - im Folgenden, wenn auf diese Kombination Bezug genommen wird, vereinfacht als Reaktor bezeichnet - erläutert.

Die Ausgangsstoffe, wie beispielsweise Erdgas und Sauerstoff, werden dabei getrennt aufgeheizt, üblicherweise bis zu 600 °C. In einer Mischzone werden die Reaktanden intensiv vermischt und nach Durchströmen eines Brennerblocks in einem Feuerraum zur exothermen Reaktion gebracht. Der Brennerblock besteht in diesen Fällen aus einer bestimmten Anzahl aus parallelen Kanälen, in denen die Strömungsgeschwindigkeit der zündfähigen Sauerstoff/Erdgas-Mischung höher ist als die Flammengeschwindigkeit (Reaktionsgeschwindigkeit, Umsetzungsgeschwindigkeit), um ein Durchschlagen der Flamme in den Mischraum zu verhindern. Der metallische Brennerblock wird gekühlt, um den thermischen Belastungen standzuhalten. Je nach Aufenthaltszeit im Mischraum entsteht die Gefahr der Vor- und Rückzündung aufgrund der begrenzten thermischen Stabilität der Mischungen. Hier wird der Begriff der Zündungsverzugszeit bzw. Induktionszeit gebraucht als diejenige Zeitspanne, in der eine zündfähige Mischung keine nennenswerte intrinsische thermische Veränderung durchläuft. Die Induktionszeit ist abhängig von der Art der eingesetzten Kohlenwasserstoffe, dem Mischungszustand, von Druck und Temperatur. Sie bestimmt die maximale Aufenthaltszeit der Reaktanden im Mischraum. Reaktanden wie Wasserstoff, Flüssiggas oder Leichtbenzin, deren Einsatz aufgrund von Ausbeute- und/oder Kapazitätssteigerungen im Syntheseprozess besonders wünschenswert ist, zeichnen sich durch eine vergleichsweise hohe Reaktivität und damit geringe Induktionszeit aus.

Die im derzeitigen Produktionsmaßstab eingesetzten Acetylenbrenner zeichnen sich durch ihre zylinderförmige Geometrie des Feuerraums aus. Der Brennerblock weist vorzugsweise hexagonal angeordnete Durchführungsbohrungen auf. In einer Ausführungsform sind z. B. 127 Bohrungen mit jeweils einem Innendurchmesser von 27 mm hexagonal auf einem kreisförmigen Grundquerschnitt mit Durchmesser von ca. 500 mm angeordnet. In der Regel liegen die eingesetzten Bohrungs- oder Kanaldurchmesser bei etwa 19 mm bis 27 mm. Der anschließende Feuerraum, in dem die Flamme der acetylenbildenden partiellen Oxidationsreaktion stabilisiert wird, ist ebenfalls von zylindrischem Querschnitt, er ist wassergekühlt und entspricht im Erscheinungsbild dem eines kurzen Rohres (von z. B. 180 mm bis 533 mm Durchmesser und 380 bis 450 mm Länge). In Höhe der feuerraumseitigen Oberfläche des Brennerblocks wird so genannter Hilfssauerstoff dem Reaktionsraum zugeführt. Dadurch wird für eine Flammenstabilisierung und somit für einen definierten Abstand der Flammenwurzel und damit des Reaktionsbeginns zum Reaktionsabbruch durch die Quencheinrichtung gesorgt. Der gesamte Brenner aus Brennerblock und Feuerraum wird in einen Quenchbehälter größeren Querschnitts über einen Flansch von oben eingehängt. Auf Höhe der Austrittsebene aus dem Feuerraum sind außerhalb von dessen Umfang Quenchdüsen auf einem oder mehreren Quenchverteilerringen installiert, die das Quenchmedium, z. B. Wasser oder Öl, mit oder ohne Zuhilfenahme eines Zerstäubungsmediums zerstäuben und näherungsweise senkrecht zur Hauptströmungsrichtung der den Feuerraum verlassenden Reaktionsgase eindüsen. Dieser direkte Quench hat die Aufgabe, die reagierende Strömung extrem schnell auf ca. 100 °C (Wasserquench) und 200 °C (Ölquench) abzukühlen, so dass Folgereaktionen, d. h. insbesondere der Abbau von gebildetem Acetylen, eingefroren werden. Die Reichweite und Verteilung der Quenchstrahlen ist dabei idealerweise so bemessen, dass eine möglichst homogene Temperaturverteilung in möglichst kurzer Zeit erreicht wird.

Die im aktuellen Produktionsmaßstab eingesetzten Acetylenbrenner zeichnen sich durch eine zylinderförmige Geometrie des Feuerraums aus. Die Einsatzstoffe werden über einen Diffusor vorgemischt und unter Vermeidung von Rückvermischung dem Brennerblock über hexagonal angeordnete Durchführungsbohrungen zugeführt. Bei den bekannten Verfahren erfolgt die Vormischung der Einsatzstoffe im Mischdiffusor in einem relativ großen Volumen und unter hohen Vorwärmtemperaturen.

Bei dem beschriebenen technischen Verfahren entsteht bei der Reaktion neben Acetylen im Wesentlichen Wasserstoff, Kohlenmonoxid und Ruß. Reaktionsbeding besteht das so gebildete Prozessgas außerdem aus substituierten Acetylenen und BTX-Aromaten (BTX - Benzol, Toluol und Xylole). Bei den BTX-Aromaten entfällt der Hauptteil auf Benzol, Toluol, Xylolisomere, Styrol und Inden. Acetylen neigt selbst bei niedrigen Temperaturen zum schlagartigen Selbstzerfall. Aus Sicherheitsgründen dürfen Acetylenpartialdrücke von 1,4 bar ohne weitere sicherheitstechnische Maßnahmen nicht überschritten werden. Eine Trennung durch destillative Verfahren ist somit ausgeschlossen. Zum Abtrennen von Acetylen aus Gasgemischen, insbesondere solchen, die durch thermische oder elektrische Spaltung von Kohlenwasserstoffen mit oder ohne Sauerstoff erhalten wurden, ist bekannt, Acetylen aus acetylenhaltigen Gasen dadurch zu gewinnen, dass man diese Gase mit einem Lösungsmittel, das eine hohe Löslichkeit für Acetylen besitzt, wäscht und dann aus der acetylenhaltigen Lösung durch mehrstufige Anwendung von Unterdruck oder erhöhter Temperatur oder durch beide Maßnahmen das reine oder stark angereicherte Acetylen ausgast. Die Löslichkeit von Acetylen in den Lösungsmitteln liegt bei Normalbedingungen zwischen 15 und 35 m³ / m³ Absorbens. Wegen der verhältnismäßig geringen Löslichkeit von Acetylen ist dies nur bei hohen Drücken wirtschaftlich, weshalb das Prozessgas komprimiert werden muss. Die Obergrenze ist limitiert durch den maximalen Partialdruck von Acetylen von 1,4 bar aufgrund der Explosionsgefahr.

Die Wirtschaftlichkeit eines derartigen Verfahrens wird dadurch in Frage gestellt, dass es Schwierigkeiten bereitet, das Lösungsmittel in der Ausgasungsstufe vollständig vom Acetylen zu befreien. Senkt man zur Erreichung dieses Zieles den Druck zu stark ab, z. B. unter 0,1 bar, so wird der Aufwand für die Verdichtung des ausgegasten Acetylens zu groß. Eine starke Erwärmung der Waschflüssigkeit, beispielsweise über 100 °C, ist kostspielig und überdies mit der Gefahr einer Zersetzung der Waschflüssigkeit und des gelösten Gases verbunden. Es ist weiter bekannt, dass man Acetylen aus Gasgemischen sehr einfach und unter wirtschaftlich günstigen Bedingungen gewinnen kann, wenn man eine Waschflüssigkeit verwendet, die neben Wasser mehr als 50 % eines organischen Lösungsmittels enthält, das Acetylen gut löst, mit Wasser mischbar ist und einen höheren Siedepunkt als Wasser besitzt. Aus derartigen Waschflüssigkeiten lässt sich das ausgewaschene Acetylen sehr leicht und vollständig dadurch ausgasen, dass man bei wirtschaftlich tragbarem Unterdruck, wie beispielsweise ungefähr 0,2 bis 0,5 bar, einen Teil des Wassers aus der Waschflüssigkeit verdampft und die Waschflüssigkeit in einer Gegenstromkolonne durch die aufsteigenden Dämpfe vom Acetylen befreit. Die Mitverwendung von Wasser bietet den Vorteil, dass man die durch Sättigung des vom Acetylen befreiten Gases und des reinen bzw. angereicherten Acetylens mit dem Dampf des Lösungsmittels verursachten Verluste vermeiden kann, indem man diese Gase mit einer kleinen Menge Wasser wäscht, das diese Lösungsmittel entfernt. Die dabei erhaltenen wässrigen Lösungen des Lösungsmittels können der Hauptmenge des organischen Lösungsmittels zugesetzt werden, um dessen Wassergehalt auf der gewünschten Höhe zu halten. Unter den als organischen Lösungsmitteln geeigneten Substanzen sind beispielsweise Butyrolacton, N-Methyl-2-pyrrolidon, Methanol, Ammoniak oder Dimethylformamid einsetzbar.

Die Kinetik der Acetylenbildung führt immer zu der Bildung von substituierten Acetylenen als Nebenprodukte, hauptsächlich Diacetylen, Methylacetylen und Vinylacetylen. Diese Komponenten polymerisieren sehr schnell und müssen so schnell wie möglich aus dem Prozessgas entfernt werden. Da sie im Absorbens viel besser löslich sind als Acetylen, ist es ausreichend, das Prozessgas mit einer kleinen Lösungsmittelmenge vorzuwaschen, bevor es in die Acetylengewinnungsstufen eintritt.

Das Absorbens N-Methyl-2-pyrrolidon wird eingesetzt, um das Prozessgas in drei Ströme aufzutrennen. Substituierte Acetylene und aromatische Verbindungen, die die am besten löslichen Bestandteile des Spaltgases sind, das Wertprodukt Acetylen, das schlechter löslich als die substituierten Acetylene ist, aber deutlich bessere Lösungseigenschaften hat als die übrigen Bestandteile des Spaltgases, das sogenannte Armgas, das hauptsächlich Wasserstoff und Kohlenmonoxid umfasst.

Im Vorwäscher wird üblicherweise das Prozessgas mit einer kleinen Menge Lösungsmittel in Kontakt gebracht, um nahezu alle aromatische Verbindungen sowie C4 und substituierte Acetylene, außer Vinylacetylen, abzutrennen. Im Hauptwäscher wird das Gas mit einer deutlich größeren Menge an N-Methyl-2-pyrrolidon in Kontakt gebracht, was das Acetylen, die verbliebenen substituierten Acetylene und etwas Kohlenmonoxid löst. Das Armgas verlässt die Kolonne am Kopf. Die N-Methyl-2-pyrrolidonlösung wird in mehreren Schritten, in denen der Druck reduziert und die Temperatur erhöht wird, entgast. Der Stripper arbeitet leicht über Umgebungsdruck und -temperatur. In dieser Kolonne wird das beladene Absorbens im Gegenstrom mit dem Gas aus dem nachfolgenden Entgasungsschritt in Kontakt gebracht. Hierzu wird der Brüdenstrom aus der Vakuumkolonne vor Eintritt in den Stripper mittels Vakuummaschine auf Betriebsdruck im Stripper gebracht. Auf diese Weise wird Kohlendioxid, welches das am schlechtesten lösliche Gas ist, am Kopf des Strippers freigesetzt. Das Kohlendioxid wird zur Saugseite des Kompressors zurückgeführt. Das Wertprodukt Acetylen wird als Seitenstrom des Strippers abgezogen. Die N-Methyl-2-pyrrolidonlösung wird anschließend in zwei weiteren Stufen bei 110-120 °C vollständig entgast, zunächst bei Atmosphärendruck und anschließend bei kleinerem Druck. Vinylacetylen, Methylacetylen und überschüssiges Prozesswasser werden als Seitenabzug aus der Vakuumkolonne entfernt. Der Wassergehalt des Lösungsmittels wird kontrolliert durch die Verdampferleistung in der Vakuumkolonne. Im Sumpf der Kolonne ist die Entgasung abgeschlossen und das Lösungsmittel wird gekühlt und wieder dem Hauptwäscher zugeführt.

Die kleine Menge an Lösungsmittel aus dem Vorwäscher wird mit Rohsynthesegas gestrippt, um gelöstes Acetylen auszutreiben und den Brüdenstrom zur Saugseite des Kompressors zurückzuführen. Das Lösungsmittel wird anschließend im Vakuumstripper vollständig entgast, wobei der gasförmige Entnahmestrom aus der Vakuumkolonne, der das überschüssige Prozesswasser sowie einige substituierte Acetylene enthält, im Gegenstrom zum Lösungsmittel strömt. Der Brüden des Vakuumstrippers enthält die höheren Acetylene, Wasser und etwas N-Methyl-2-pyrrolidondampf. In einer Seitenkolonne wird das N-Methyl-2-pyrrolidon mit wenig Wasser zurückgewaschen und das Wasser-N-Methyl-2-pyrrolidon-Gemisch dem Hauptlösungsmittelstrom zugeführt. Das Gas wird durch Direktkontakt mit Wasser im Mischkondensator gekühlt, um den Großteil des Wasserdampfes auszukondensieren. Die substituierten Acetylene werden vor der Vakuumpumpe verdünnt mit z.B. Erdgas oder Armgas. Die verdünnten substituierten Acetylene, die an dieser Stelle bei leichtem Überdruck vorliegen, können als Verbrennungsgas genutzt werden. Um möglichst wenig Wasserdampf in die Verbrennung zu geben, werden die substituierten Acetylene in einem nachgeschalteten Gaskühler hinter der Vakuumpumpe erneut abgekühlt, um Wasserdampf auszukondensieren. Dazu wird das Gas im Gegenstrom mit Kreislaufwasser direktgekühlt. Anschließend wird es mit z. B. Erdgas oder Armgas weiter verdünnt und zur Verbrennung gegeben. Um den Polymergehalt des Lösungsmittels zu minimieren, werden etwa 2 % der Lösungsmittelmenge des Vakuumstripperkreislaufs kontinuierlich abgezogen und bei erniedrigtem Druck abdestilliert.

Trotz der durch diese Verfahren und Vorrichtungen bewirkten Vorteile besteht nach wie vor ein Verbesserungspotenzial. So werden beim bestehenden Verfahren zur Acetylensynthese durch partielle Oxidation nach Sachsse-Bartholomé mit Wasserquench und Ölquench die Acetylene und substituierten Acetylene aus dem Lösemittel N-Methyl-2-pyrrolidon zwecks Acetylengewinnung und Lösungsmittelregeneration abgetrennt. Die Desorption der im Vor-und Hauptwäscher gelösten Prozessgaskomponenten und die Regenerierung der N-Methyl-2-pyrrolidonkreisläufe erfolgt nach dem Stand der Technik im Vakuum bei ca. 150 bis 500 mbar (abs.), meist bei 200 mbar (abs.), in der Vakuumkolonne und im Gasweg für die substituierten Acetylene, der unter anderem einen Vakuumstripper, Gas-Wäscher für N-Methyl-2-pyrrolidonund einen Mischkondensator aufweist. Dieses Vorgehen führt zu hohen Investitionskosten bei Maschinen und Apparaten in diesem Anlagenteil, d.h. für die Apparate zur Unterdruckerzeugung in der Anlage.

Es ist daher Aufgabe der vorliegenden Erfindung, eine Vorrichtung und ein Verfahren zum Herstellen von Acetylen und Synthesegas anzugeben, das die oben beschriebenen Nachteile zumindest weitgehend vermeidet und die insbesondere eine einfachere Betriebsweise bei Normaldruck bzw. leichtem Überdruck und somit eine vakuumfreie Betriebsweise erlauben.

Eine erfindungsgemäße Vorrichtung zum Herstellen von Acetylen und Synthesegas durch partielle Oxidation von Kohlenwasserstoffen mit Sauerstoff, umfasst einen Reaktor, wobei der Reaktor einen Brennerblock mit einem Feuerraum zur Herstellung einer Zusammensetzung Z1 enthaltend zumindest Acetylen und substituiertes Acetylen aufweist. Die Vorrichtung umfasst weiterhin einen ersten Wäscher, der zum Versetzen der Zusammensetzung Z1 mit einem Lösungsmittel unter Erhalt einer Zusammensetzung Z2 ausgebildet ist. Die Vorrichtung umfasst weiterhin einen zweiten Wäscher, der zum Versetzen der Zusammensetzung Z2 mit dem Lösungsmittel unter Erhalt einer Zusammensetzung Z3 ausgebildet ist. Die Vorrichtung umfasst weiterhin einen ersten Stripper, der zum Strippen der Zusammensetzung Z3 unter Erhalt einer Zusammensetzung Z4 enthaltend das substituierte Acetylen, Acetylen und das Lösungsmittel und zum Abtrennen des Acetylens ausgebildet ist. Die Vorrichtung umfasst weiterhin eine erste Kolonne, die zum teilweisen Entgasen der Zusammensetzung Z4 bei einem Druck von 1,0 bar bis 1,5 bar unter Erhalt einer Zusammensetzung Z5, einer Zusammensetzung Z10 und eines ersten Anteils A1 des Lösungsmittels ausgebildet ist. Die Vorrichtung umfasst weiterhin einen zweiten Stripper, dem zum Strippen einer Zusammensetzung Z9 die Zusammensetzung Z5 unter Erhalt eines zweiten Anteils A2 des Lösungsmittels und einer Zusammensetzung Z6 zuführbar ist. Die Vorrichtung umfasst weiterhin einen dritten Stripper, der zum Strippen des Lösungsmittels aus dem ersten Wäscher unter Erhalt einer Zusammensetzung Z9 ausgebildet ist, wobei der dritte Stripper zum Zuführen der Zusammensetzung Z9 zu dem zweiten Stripper mit dem zweiten Stripper verbunden ist. Die Vorrichtung umfasst weiterhin eine Vorrichtung zum Versetzen der Zusammensetzung Z5 mit einem Verdünnungsgas, die zwischen der ersten Kolonne und dem zweiten Stripper angeordnet ist. Die Vorrichtung umfasst weiterhin eine zweite Kolonne, die zum Versetzen der Zusammensetzung Z6 mit Wasser unter Erhalt einer Zusammensetzung Z7 enthaltend einen dritten Anteil A3 des Lösungsmittels und Wasser und unter Erhalt einer Zusammensetzung Z8 enthaltend das substituierte Acetylen ausgebildet ist. Die Vorrichtung umfasst weiterhin einen Mischkondensator, der zum Versetzen der Zusammensetzung Z8 mit Wasser unter Erhalt einer Zusammensetzung Z11 enthaltend das substituierte Acetylen ausgebildet ist.

Unter substituiertem Acetylen ist im Rahmen der vorliegenden Erfindung Acetylen zu verstehen, bei dem ein Wasserstoffatom substituiert ist. Im Rahmen der vorliegenden Erfindung handelt es sich dabei insbesondere um monosubstituiertes Acetylen. Das Wasserstoffatom ist insbesondere durch einen Alkyl-, Alkenyl- oder Alkinylrest substituiert. Der Alkylrest kann insbesondere Methyl sein, der Alkenylrest kann insbesondere Vinyl sein und der Alkinylrest kann insbesondere Ethinyl sein. Somit handelt es sich bei dem substituierten Acetylen der vorliegenden Erfindung überwiegend, aber nicht ausschließlich, um Methylacetylen, Vinylacetylen und Diacetylen.

Unter einem Lösungsmittel ist im Rahmen der vorliegenden Erfindung grundsätzlich jedes Lösungsmittel zu verstehen, das eine Löslichkeit für Acetylen und substituiertes Acetylen aufweist. Insbesondere sind im Rahmen der vorliegenden Erfindung organische Lösungsmittel einsetzbar, die mit Wasser mischbar sind und einen höheren Siedepunkt als Wasser aufweisen.

Unter Strippen ist im Rahmen der vorliegenden Erfindung ein Trennverfahren, insbesondere ein physikalisches Trennverfahren, zu verstehen, bei dem Stoffe aus einer flüssigen Phase durch Desorptionsvorgänge in die Gasphase überführt werden. Dabei wird die Flüssigphase im Gegenstromprinzip mit einem Gas, dem sogenannten Strippgas, in Kontakt gebracht. Unter Desorption ist dabei ein Vorgang zu verstehen, bei dem in der Flüssigkeit gebundene Gaskomponenten aus dieser Flüssigkeit durch Druckverminderung und/oder Temperaturerhöhung und/oder Partialdruckverringerung durch ein Strippgas ausgetrieben werden. Die Desorption stellt somit den Umkehrvorgang der Absorption dar.

Entsprechend ist im Rahmen der vorliegenden Erfindung unter einem Stripper eine Vorrichtung zu verstehen, die zum Strippen ausgebildet und geeignet ist. Der Stripper kann beispielsweise in Form einer Kolonne ausgebildet sein. Über eine Düse oder einen Flüssigkeitsverteiler wird die geförderte Flüssigkeit am Kopf bzw. Kopfende des Strippers fein verteilt, so dass diese in der Kolonne in den Sumpf rieselt, insbesondere über Füllkörper, strukturierte Packungen oder Böden. Im Gegenstrom wird das Strippgas durch den Stripper gefördert. Die Füllkörper dienen dazu, die Flüssigkeit fein zu verteilen und somit die Phasengrenzfläche zu maximieren. Füllkörper dienen zur Vergrößerung der Wirkungs-Oberfläche bei gleichzeitig geringem Strömungswiderstand. Füllkörper werden je nach Einsatzzweck aus verschiedenen Materialien hergestellt, wie beispielsweise rostfreiem Stahl, Kunststoff oder aus Keramik. Im Rahmen der vorliegenden Erfindung werden bevorzugt Füllkörper aus rostfreiem Stahl eingesetzt.

Unter einer Kolonne ist im Rahmen der vorliegenden Erfindung ein verfahrenstechnischer Apparat in Form einer hohlen, schlanken Säule mit internen Einbauten zu verstehen, welche auf Grund des Einsatzzweckes oder der Betriebsbedingungen gefordert werden. Im Rahmen der vorliegenden Erfindung dient die Kolonne dazu, Stoffgemische durch diverse thermische Verfahren zu trennen. Hierzu werden physikalische Eigenschaften und Gleichgewichtszustände zwischen unterschiedlichen Phasen genutzt. In der Kolonne werden zwei Phasen im Gegenstrom direkt miteinander in Kontakt gebracht. Einbauten in der Kolonne dienen dem erhöhten Stoffaustausch und Energieaustausch zwischen den Phasen oder der Vermeidung einer Rückvermischung.

Unter einem Mischkondensator ist im Rahmen der vorliegenden Erfindung eine Vorrichtung zu verstehen, die zum Kondensieren von Gasen oder Dämpfen im direkten Kontakt mit Kühlwasser ausgebildet und geeignet ist. Kühlwasser und Kondensat werden dabei vermischt.

Die im Rahmen der vorliegenden Erfindung genannten Drücke sind absolute Drücke, sofern nicht explizit etwas anderes beschrieben ist. Die für den Druck angegebene Einheit ist "bar", "mbar" und kann zur Verdeutlichung des absoluten Drucks auch als "bar (abs.)" oder "mbar (abs.)" angegeben sein. In der Regel wird dabei verkürzend nur die Einheit "bar" oder "mbar" genannt.

Das Verdünnungsgas kann ausgewählt sein aus der Gruppe bestehend aus: H₂, N₂, CO₂, NH₃, Armgas, Erdgas. Beispielsweise wird bei Wegfall des Energieeintrages in den Sumpf der ersten Kolonnen und damit der fehlenden Verdampfung von Wasser und N-Methyl-2-pyrrolidon das Verdünnungsgas direkt in die erste Kolonne zur Druckhaltung in der ersten Kolonne dosiert. Damit wird gewährleistet, dass die Konzentration des Gases der substituierten Acetylene im Seitenabzug unterhalb der Zerfallsgrenze liegt.

Der aus dem zweiten Stripper erhaltene zweite Anteil A2 des Lösungsmittels ist dem ersten und/oder zweiten Wäscher zuführbar. Damit wird die Menge des eingesetzten Lösungsmittels reduziert, da das Lösungsmittel durch die erfindungsgemäße Vorrichtung zu einem Großteil zurückgewonnen wird.

Der aus der ersten Kolonne erhaltene erste Anteil A1 des Lösungsmittels ist dem ersten und/oder zweiten Wäscher zuführbar. Damit wird die Menge des eingesetzten Lösungsmittels reduziert, da das Lösungsmittel durch die erfindungsgemäße Vorrichtung zu einem Großteil zurückgewonnen wird.

Die erste Kolonne kann zum Abführen der Zusammensetzung Z5 einen Seitenabzug aufweisen. Bevorzugt grenzt die Vorrichtung zum Versetzen der Zusammensetzung Z5 mit einem Verdünnungsgas an den Seitenabzug an. Durch den dampfförmigen Seitenabzug werden dabei das angereicherte Vinylacetylen, die Acetylenverluste, sowie N-Methyl-2-pyrrolidon- und Wasserdampf abgezogen. Dieser Strom, der laut Stand der Technik bei etwa 200 mbar (abs.) und etwa 100 °C vorliegt, verändert sich in der Zusammensetzung nur gering und liegt bei der erfindungsgemäßen Vorrichtung bei etwa 1,4 bar (abs.) und ca. 150°C vor. Für den Fall, dass dieser Strom trocken ist, berechnet sich ein kritischer Deflagrationsdruck von < 0,5 bar (abs.) und wird somit von dem Betriebsdruck in der Kolonne überschritten. Daher muss der Strom verdünnt werden auf einen kritischen Deflagrationsdruck von > 1,5 bar (abs.). Das erfindungsgemäße Konzept sieht vor, dass ein Verdünnungsgas zwischen der ersten Kolonne und dem zweiten Stripper zu dem Seitenabzug dosiert wird. Durch eine Anordnung der Vorrichtung zum Versetzen der Zusammensetzung Z5 mit einem Verdünnungsgas in der Nähe des Seitenabzugs lässt sich der Druck besonders gut und schnell steuern.

Eine Temperatur in dem Mischkondensator kann zum Verhindern eines Ausfalls von Naphthalin eingestellt sein. Beispielsweise ist die Temperatur in dem Mischkondensator zwischen 40 °C und 80 °C einzustellen. Vorzugsweise beträgt die Temperatur 60 °C bis 70 °C, beispielsweise 65 °C.

Der zweite Stripper kann zum Entgasen der Zusammensetzung Z9 bei einem Druck von 1,0 bar bis 1,4 bar betrieben werden. Somit erlaubt die erfindungsgemäße Vorrichtung eine vakuumfreie Betriebsweise. Das wirtschaftliche Potenzial liegt in der Reduzierung der Investitionskosten durch Wegfall von Vakuumeinheiten inklusive der peripheren Kühleinrichtungen durch ein sicherheitstechnisch realisierbares Verfahrenskonzept.

Das Lösungsmittel kann N-Methyl-2-pyrrolidon sein. Alternativ können andere organische Lösungsmittel verwendet werden, wie beispielsweise Butyrolacton, Methanol, Ammoniak oder Dimethylformamid.

Ein erfindungsgemäßes Verfahren zum Herstellen von Acetylen und Synthesegas durch partielle Oxidation von Kohlenwasserstoffen mit Sauerstoff, umfasst die folgenden Schritte, bevorzugt in der angegebenen Reihenfolge:
- partielles Oxidieren von Kohlenwasserstoffen mit Sauerstoff zum Herstellen einer Zusammensetzung Z1 enthaltend zumindest Acetylen und substituiertes Acetylen,
- Versetzen der Zusammensetzung Z1 mit einem Lösungsmittel unter Erhalt einer Zusammensetzung Z2,
- Versetzen der Zusammensetzung Z2 mit dem Lösungsmittel unter Erhalt einer Zusammensetzung Z3,
- Strippen der Zusammensetzung Z3 unter Erhalt einer Zusammensetzung Z4 enthaltend das substituierte Acetylen, Acetylen und das Lösungsmittel und zum Abtrennen des Acetylens aus der Zusammensetzung Z3,
- teilweises Entgasen der Zusammensetzung Z4 bei einem Druck von 1,0 bar bis 1,5 bar unter Erhalt einer Zusammensetzung Z5 und eines ersten Anteils A1 des Lösungsmittels,
- Strippen einer Zusammensetzung Z9 mit der Zusammensetzung Z5 unter Erhalt eines zweiten Anteils A2 des Lösungsmittels und einer Zusammensetzung Z6,
- Strippen des nach der Herstellung der Zusammensetzung Z1 erhaltenen Lösungsmittels unter Erhalt der Zusammensetzung Z9,
- Versetzen der Zusammensetzung Z5 mit einem Verdünnungsgas,
- Versetzen der Zusammensetzung Z6 mit Wasser unter Erhalt einer Zusammensetzung Z7 enthaltend einen dritten Anteil A3 des Lösungsmittels und Wasser und unter Erhalt einer Zusammensetzung Z8 enthaltend das substituierte Acetylen, und
- Versetzen der Zusammensetzung Z8 mit Wasser unter Erhalt einer Zusammensetzung Z11 enthaltend das substituierte Acetylen.

Das Verdünnungsgas kann ausgewählt sein aus der Gruppe bestehend aus: H₂, N₂, CO₂, NH₃, Armgas, Erdgas. Beispielsweise wird bei Wegfall des Energieeintrages in den Sumpf der ersten Kolonnen und damit der fehlenden Verdampfung von Wasser und N-Methyl-2-pyrrolidon das Verdünnungsgas direkt in die erste Kolonne zur Druckhaltung in der ersten Kolonne dosiert. Damit wird gewährleistet, dass die Konzentration des Gases der substituierten Acetylene im Seitenabzug unterhalb der Zerfallsgrenze liegt.

Der erhaltene zweite Anteil A2 des Lösungsmittels kann der Zusammensetzung Z1 und/oder Z2 zugeführt werden. Damit wird die Menge des eingesetzten Lösungsmittels reduziert, da das Lösungsmittel durch die erfindungsgemäße Vorrichtung zu einem Großteil zurückgewonnen wird.

Der erhaltene erste Anteil A1 des Lösungsmittels kann der Zusammensetzung Z1 und/oder Z2 zugeführt werden. Damit wird die Menge des eingesetzten Lösungsmittels reduziert, da das Lösungsmittel durch die erfindungsgemäße Vorrichtung zu einem Großteil zurückgewonnen wird.

Eine Temperatur der Zusammensetzung Z11 kann zum Verhindern eines Ausfalls von Naphthalin eingestellt werden. Beispielsweise ist die Temperatur in dem Mischkondensator zwischen 40 °C und 80 °C einzustellen. Vorzugsweise beträgt die Temperatur 60 °C bis 70 °C, beispielsweise 65 °C.

Die Zusammensetzung Z9 kann bei einem Druck von 1,0 bar bis 1,4 bar entgast werden. Somit erlaubt das erfindungsgemäße Verfahren eine vakuumfreie Betriebsweise. Das wirtschaftliche Potenzial liegt in der Reduzierung der Investitionskosten durch Wegfall von Vakuumeinheiten inklusive der peripheren Kühleinrichtungen durch ein sicherheitstechnisch realisierbares Verfahrenskonzept.

Das Lösungsmittel kann N-Methyl-2-pyrrolidon sein. Alternativ können andere organische Lösungsmittel verwendet werden, wie beispielsweise Butyrolacton, Methanol, Ammoniak oder Dimethylformamid.

Ein Grundgedanke der vorliegenden Erfindung ist eine Strippung des beladenen Lösungsmittels, das bevorzugt N-Methyl-2-pyrrolidons ist, unter Normaldruck bzw. leichtem Überdruck, wie beispielsweise ungefähr 1,1-1,4 bar (abs.), vorzugsweise 1,25 bar (abs.), und somit eine vakuumfreie Betriebsweise. Das wirtschaftliche Potenzial der Erfindung liegt in der Reduzierung der Investitionskosten durch Wegfall von zwei Vakuumeinheiten inklusive der peripheren Kühleinrichtungen durch ein sicherheitstechnisch realisierbares Verfahrenskonzept. Bei der Bearbeitung des Neuverfahrens hat sich von der Verschaltung der einzelnen Apparate bis auf den Wegfall der Vakuumaggregate samt den folgenden Kühleinrichtungen nichts verändert. Die prinzipielle Stromführung bleibt fast vollständig erhalten, wobei eine Anpassung der Betriebsbedingungen Druck und Temperatur an die Normaldruckfahrweise erfolgt. Lediglich die Verdünnung des die substituierten Acetylene enthaltenden Gases zur Absenkung des Partialdrucks und damit die Stabilisierung des die substituierten Acetylene enthaltenden Gases erfolgt nicht mehr nach Kühlung im Mischkondensator und vor der Verdichtung, sondern zwischen dem Gasabzug aus der Seite der ersten Kolonne und dem zweiten Stripper, bevorzugt direkt nach dem Seitenabzug. Zielvorgabe ist der Abgabedruck des die substituierten Acetylene enthaltenden Gases mit 1,2 bar (abs.). Die Drücke in den davorliegenden Apparaten sind über die erwartete Druckverlustkette definiert.

Die wichtigste neue Herausforderung aus der Verfahrensänderung ist die Gefährdungseinschätzung des neuen Konzeptes beim Umgang mit den stofflichen Gefährdungen von Acetylen und den substituierten Acetylenen durch deren Neigung zum explosiven Selbstzerfall. Erfinderisches Merkmal des erfindungsgemäßen Verfahrens ist ein sicherheitstechnisch einwandfrei realisierbares Verfahrenskonzept. Zur sicherheitstechnischen Bewertung einzelner Prozessströme wurde der geplante Prozessdruck mit dem entsprechenden kritischen Deflagrationsdruck verglichen. Als Basis sind die Berechnungen nach Le Chatelier - ohne Wasserdampfpartialdruck - zugrunde gelegt.

In der ersten Kolonne wird das mit Acetylen und Vinylacetylen (Hauptbestandteile) beladene N-Methyl-2-pyrrolidon regeneriert, d. h. durch Energieeintrag in den Sumpfverdampfer wird Strippdampf erzeugt, welcher das beladene N-Methyl-2-pyrrolidon über Kopf- und Seitenabzug von den absorbierten Komponenten befreit und auf einen vorgegebenen Wassergehalt einstellt. Durch den dampfförmigen Seitenabzug werden dabei das angereicherte Vinylacetylen, die Acetylenverluste, sowie N-Methyl-2-pyrrolidon- und Wasserdampf abgezogen. Dieser Strom, der laut Stand der Technik bei etwa 200 mbar (abs.) und etwa 100 °C vorliegt, verändert sich in der Zusammensetzung nur gering und liegt dann bei etwa 1,4 bar (abs.) und ca. 150°C vor. Für den Fall, dass dieser Strom trocken ist, berechnet sich ein kritischer Deflagrationsdruck von < 0,5 bar (abs.) und wird somit von dem Betriebsdruck in der Kolonne überschritten. Daher muss der Strom verdünnt werden auf einen kritischer Deflagrationsdruck von > 1,5 bar (abs.). Das erfindungsgemäße Konzept sieht vor, dass ein Verdünnungsgas zwischen erster Kolonne und zweitem Stripper zu dem Seitenabzug dosiert wird. Der phlegmatisierende Einfluss von Verdünnungsgasen steigt aufgrund ihrer Wärmekapazität in der Reihenfolge H₂ < N₂ < CO₂ < NH₃. Als Verdünnungsgas können u. a. folgende Gase eingesetzt werden: H₂, N₂, CO₂, NH₃, Armgas, Erdgas und andere. Das erfindungsgemäße Konzept sieht vor, dass beim Wegfall des Energieeintrages in den Kolonnensumpf und damit der fehlenden Verdampfung von Wasser und N-Methyl-2-pyrrolidon, das Verdünnungsgas direkt in die Kolonne zur Druckhaltung in der Kolonne dosiert wird. Damit wird gewährleistet, dass die Konzentration des die substituierten Acetylene enthaltenden Gases im Seitenabzug unterhalb der Zerfallsgrenze liegt.

Entsprechend zur ersten Kolonne wird im zweiten Stripper das beladene N -Methylpyrrolidon aus dem ersten Wäscher regeneriert, d. h. neben der Eigendampferzeugung durch Energieeintrag in den Sumpfverdampfer wird der gasförmige Seitenabzug der ersten Kolonne in den Sumpf eingespeist. Der über Kopf abgezogene Gasstrom enthält neben N-Methyl-2-pyrrolidon- und Wasserdampf die höchste Konzentration des die substituierten Acetylene enthaltenden Gases im Prozess. Er wird durch Zugabe von einer vorgegebenen Menge Verdünnungsgas verdünnt. Der für den (theoretisch) trockenen Strom berechnete kritische Deflagrationsdruck von > 1,5 bar (abs.) liegt oberhalb des Betriebsdruckes der Kolonne. Für die im Gasweg des die substituierten Acetylene enthaltenden Gases folgende zweite Kolonne und den Mischkondensator ergeben sich durch die Verdünnung annähernd gleiche Differenzen zwischen berechnetem kritischem Deflagrationsdruck der Gasströme und Prozessdruck. Die Fahrweise bei Normaldruck macht die Gasmaschine für das die substituierten Acetylene enthaltende Gas sowie den Gaskühler für das die substituierten Acetylene enthaltende Gas überflüssig. Dadurch verlagert sich die kritische Stelle für Naphthalin in den Mischkondensator. Sollte bei gegebenem Druck und Temperatur mehr Naphthalin vorhanden sein, als im Gasstrom zu transportieren ist, d. h. wenn der Partialdruck höher ist als der Sublimationsdruck, so resublimiert Naphthalin und fällt als Feststoff aus. Durch entsprechende Temperaturführung, insbesondere Temperaturen zwischen 40 °C und 80 °C und vorzugsweise zwischen 60 °C und 70 °C, beispielsweise 65 °C, kann der Ausfall von Naphthalin vermieden werden. Hinter dem Mischkondensator wird durch Verdünnen mit z. B. Erdgas oder Armgas der Abstand vom Taupunkt vergrößert, so dass Kondensation auf dem Weg zur Fackel vermieden werden kann.

Weitere optionale Einzelheiten und Merkmale der vorliegenden Erfindung ergeben sich aus der nachfolgenden Beschreibung bevorzugter Ausführungsbeispiele, welche schematisch in der Zeichnung dargestellt sind.

Es zeigt:

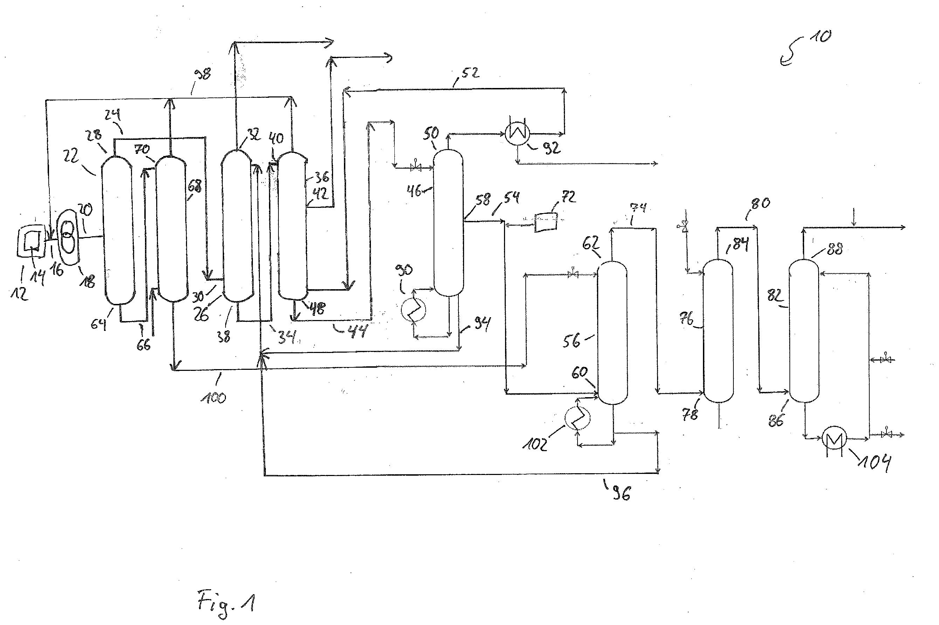

- Figur 1: ein verfahrenstechnisches Schaltbild einer erfindungsgemäßen Vorrichtung zum Herstellen von Acetylen und Synthesegas durch partielle Oxidation von Kohlenwasserstoffen mit Sauerstoff.

### Ausführungsformen der Erfindung

Figur 1 zeigt ein Schaltbild einer erfindungsgemäßen Vorrichtung 10 zum Herstellen von Acetylen und Synthesegas durch partielle Oxidation von Kohlenwasserstoff mit Sauerstoff. Die Vorrichtung 10 umfasst einen Reaktor 12. Der Reaktor 12 weist einen Brennerblock 14 mit einem nicht näher gezeigten Feuerraum zur Acetylenherstellung auf. Dem Reaktor 12 ist zum Herstellen von Acetylen und Synthesegas durch partielle Oxidation ein Kohlenwasserstoff enthaltender Strom sowie ein Sauerstoff enthaltender Strom zuführbar.

Der Reaktor 12 und insbesondere der Brennerblock 14 sind über eine Leitung 16 mit einem Kompressor 18 verbunden. Der Kompressor 18 ist über eine Leitung 20 mit einem ersten Wäscher 22 verbunden. Der erste Wäscher 22 kann beispielsweise als Vorwäscher ausgebildet sein. Der erste Wäscher 22 ist über eine Leitung 24 mit einem zweiten Wäscher 26 verbunden. Der zweite Wäscher 26 kann beispielsweise als Hauptwäscher ausgebildet sein. Die Leitung 24 erstreckt sich von einem Kopfende 28 des ersten Wäschers 22 zu einem unteren seitlichen Ende 30 des zweiten Wäschers 26. Der zweite Wäscher 26 ist so ausgebildet, dass an einem Kopfende 32 des zweiten Wäschers 26 ein so genanntes Armgas, das hauptsächlich Wasserstoff und Kohlenmonoxid umfasst, entnehmbar oder abführbar ist. Der zweite Wäscher 26 ist über eine Leitung 34 mit einem ersten Stripper 36 verbunden.

Der erste Stripper 36 ist bei dem gezeigten Ausführungsbeispiel als ein Gegenstromstripper ausgebildet. Die Leitung 34 erstreckt sich von einem unteren Ende 38 des zweiten Wäschers 26 zu einem Kopfende 40 des ersten Strippers 36. Der erste Stripper 36 ist so ausgebildet, dass in dem ersten Stripper 36 Acetylen abtrennbar ist. Das Acetylen ist beispielsweise an einer Seite 42 des ersten Strippers 36 als Seitenstrom abziehbar.

Der erste Stripper 36 ist über eine Leitung 44 mit einer ersten Kolonne 46 verbunden. Die Leitung 44 erstreckt sich von einem unteren Ende 48 des ersten Strippers 36 zu einem Kopfende 50 der ersten Kolonne 46. Die Leitung 44 geht beispielsweise an einem tiefsten Punkt des ersten Strippers 36 ab und führt zu einem seitlichen Eingang an dem Kopfende 50 der ersten Kolonne 46. Die erste Kolonne 46 ist zusätzlich über eine Leitung 52 mit dem ersten Stripper 36 verbunden. Die Leitung 52 erstreckt sich von dem Kopfende 50 der ersten Kolonne 46 zu dem unteren Ende 48 des ersten Strippers 36. Die Leitung 52 geht beispielsweise an einem höchsten Punkt der ersten Kolonne 46 ab und führt zu einem seitlichen Eingang an dem unteren Ende 48 des ersten Strippers 36.

Die erste Kolonne 46 ist über eine Leitung 54 mit einem zweiten Stripper 56 verbunden. Die Leitung 54 erstreckt sich von einem Seitenabzug 58 der ersten Kolonne 46 zu einem unteren Ende 60 des zweiten Strippers 56. Der zweite Stripper 56 weist weiter ein Kopfende 62 auf. Ein unteres Ende 64 des ersten Wäschers 22 ist über eine Leitung 66 mit einem dritten Stripper 68 verbunden. Die Leitung 66 erstreckt sich von dem unteren Ende 64 des ersten Wäschers 22 zu einem Kopfende 70 des dritten Strippers 68. Beispielsweise geht die Leitung 66 an einem tiefsten Punkt des ersten Wäschers 22 ab.

Zwischen der ersten Kolonne 46 und dem zweiten Stripper 56 ist eine Vorrichtung 72 angeordnet. Die Vorrichtung 72 ist genauer in der Nähe des Seitenabzugs 58 der ersten Kolonne 46 angeordnet. Die Vorrichtung 72 ist zum Zuführen eines Verdünnungsgases zu der Leitung 54 ausgebildet. Das Verdünnungsgas ist ausgewählt aus der Gruppe bestehend aus H₂, N₂, CO₂, NH₃, Armgas und Erdgas. Der zweite Stripper 56 ist über eine Leitung 74 mit einer zweiten Kolonne 76 verbunden. Die Leitung 74 erstreckt sich von dem Kopfende 62 des zweiten Strippers 56 zu einem unteren Ende 78 der zweiten Kolonne 76. Beispielsweise geht die Leitung 74 an einem höchsten Punkt des zweiten Strippers 56 ab und führt zu einem seitlichen Eingang an dem unteren Ende 78 der zweiten Kolonne 76. Die zweite Kolonne 76 ist über eine Leitung 80 mit einem Mischkondensator 82 verbunden. Die Leitung erstreckt sich von einem Kopfende 84 der zweiten Kolonne 76 zu einem unteren Ende 86 des Mischkondensators 82. Beispielsweise geht die Leitung 80 von einem höchsten Punkt der zweiten Kolonne 76 ab und führt zu einem seitlichen Eingang an dem unteren Ende 86 des Mischkondensators 82. Der Mischkondensator 82 ist so ausgebildet, dass das substituierte Acetylen an einem Kopfende 88 des Mischkondensators 82 entnehmbar ist.

Nachstehend wird eine mögliche erfindungsgemäße Betriebsweise der Vorrichtung 10 beschrieben. Dem Reaktor 12 werden ein Kohlenwasserstoff enthaltender Strom sowie ein Sauerstoff enthaltender Strom zugeführt. Die beiden Ströme reagieren miteinander in dem Reaktor 12. Bei einer Reaktion des Kohlenwasserstoffs mit dem Sauerstoff in dem Reaktor 12 entsteht zu einem Großteil Acetylen. Durch die partielle Oxidation von Kohlenwasserstoff und Sauerstoff wird jedoch nicht ausschließlich Acetylen hergestellt. Die Kinetik der Acetylenbildung führt immer auch zu der Bildung von substituierten Acetylenen als Nebenprodukte. Die substituierten Acetylene umfassen hauptsächlich Diacetylen, Methylacetylen und Vinylacetylen. Genauer wird somit eine Zusammensetzung Z1 hergestellt, die zumindest Acetylen und substituiertes Acetylen aufweist. Da die substituierten Acetylene sehr schnell polymerisieren, müssen sie so schnell wie möglich aus dem Prozessgas bzw. der Zusammensetzung Z1 entfernt werden. Nachstehend werden die Ausdrücke "substituiertes Acetylen" und "substituierte Acetylene" synonym verwendet, sofern nicht anders angegeben, um auszudrücken, dass die Zusammensetzung Z1 mindestens ein substituiertes Acetylen aufweist.

Die Zusammensetzung Z1 wird über die Leitung 16 dem Kompressor 18 zugeführt und dort verdichtet. Anschließend wird die Zusammensetzung Z1 über die Leitung 20 dem ersten Wäscher 22 zugeführt. In dem ersten Wäscher 22 wird die Zusammensetzung Z1 mit einem Lösungsmittel, wie beispielsweise N-Methyl-2-pyrrolidon, versetzt. Das Lösungsmittel dient als Absorbens. Die Menge des Lösungsmittels ist dabei gering, da das substituierte Acetylen in dem Absorbens viel besser löslich ist als Acetylen. Mit anderen Worten ist es ausreichend, das Prozessgas mit einer kleinen Lösungsmittelmenge vorzuwaschen, bevor es in die nachstehend näher beschriebenen Acetylengewinnungsstufen eintritt. In dem ersten Wäscher 22 wird das Lösungsmittel N-Methyl-2-pyrrolidon eingesetzt, um alle aromatische Verbindungen sowie C4 und substituierte Acetylene, außer Vinylacetylen, abzutrennen. Genauer wird das Lösungsmittel N-Methyl-2-pyrrolidon eingesetzt, um das Prozessgas oder die Zusammensetzung Z1 in drei Ströme aufzutrennen. Der erste Strom umfasst substituierte Acetylene und aromatische Verbindungen, die die am besten löslichen Bestandteile der Zusammensetzung Z1 sind. Der zweite Strom umfasst das Wertprodukt Acetylen, das schlechter löslich ist als die substituierten Acetylene aber deutlich bessere Lösungseigenschaften hat als die übrigen Bestandteile der Zusammensetzung Z1. Der dritte Strom umfasst Rohsynthesegas, das hauptsächlich Wasserstoff und Kohlenmonoxid umfasst. In dem ersten Wäscher 22 wird das Lösungsmittel eingesetzt, um alle aromatische Verbindungen sowie C4 und das substituierte Acetylen nahezu vollständig (außer Vinylacetylen) abzutrennen.

Durch das Vorwaschen in dem ersten Wäscher 22 wird eine Zusammensetzung Z2 erhalten. Die Zusammensetzung Z2 umfasst alle nicht abgetrennten Verbindungen. Mit anderen Worten umfasst die Zusammensetzung Z2 nahezu keine aromatischen Verbindungen, nahezu keine C4-Verbindungen sowie nahezu kein substituiertes Acetylen mit Ausnahme von Vinylacetylen. Die Zusammensetzung Z2 wird dem zweiten Wäscher 26 über die Leitung 24 zugeführt. In dem zweiten Wäscher 26 wird die Zusammensetzung Z2 mit dem Lösungsmittel unter Erhalt einer Zusammensetzung Z3 versetzt. Die Menge des Lösungsmittels in dem zweiten Wäscher 26 ist dabei im Vergleich zu der Menge des Lösungsmittels in dem ersten Wäscher 22 deutlich größer. Die größere Menge des Lösungsmittels löst das Acetylen, das verbliebene substituierte Acetylen und etwas Kohlenmonoxid. Der zweite Wäscher 26 ist so ausgebildet, dass das so genannte Armgas, das hauptsächlich Wasserstoff und Kohlenmonoxid umfasst, den zweiten Wäscher 26 an dem Kopfende 32 des zweiten Wäschers 26 verlässt. Die Zusammensetzung Z3 umfasst somit gelöstes Acetylen, das verbliebene substituierte Acetylen und etwas Kohlenmonoxid.

Die Zusammensetzung Z3 wird dem ersten Stripper 36 über die Leitung 34 zugeführt. Der erste Stripper 36 ist zum Strippen der Zusammensetzung Z3 unter Erhalt einer Zusammensetzung Z4 und zum Abtrennen des Acetylens ausgebildet. In dem ersten Stripper 36 wird die Zusammensetzung Z3 gestrippt. Dabei wird die Zusammensetzung Z4 erhalten, die das substituierte Acetylen, Acetylen und das Lösungsmittel enthält. Der erste Stripper 36 arbeitet geringfügig über Umgebungsdruck und -temperatur. Dabei wird Kohlendioxid, welches das am schlechtesten lösliche Gas ist, an dem Kopfende 40 des ersten Strippers 36 freigesetzt. Das Kohlendioxid kann zur Saugseite des Kompressors 18 zurückgeführt werden. Das Acetylen wird beispielsweise an der Seite 42 des ersten Strippers 36 als Seitenstrom abgezogen.

Die Zusammensetzung Z4 wird der ersten Kolonne 46 über die Leitung 44 zugeführt. Hierbei wird dem Strom in einer Art und Weise Wärme zugeführt und der Druck erhöht, dass die Zusammensetzung Z4 in der Leitung 44 einen Druck von 7,5 bar bis 8,5 bar und eine Temperatur von 125 °C bis 135 °C, aufweist. Beispielsweise weist die Zusammensetzung Z4 in der Leitung 44 einen Druck von 8,0 bar und eine Temperatur von 130 °C auf. Die erste Kolonne 46 ist zum teilweisen Entgasen der Zusammensetzung Z4 bei einem Druck von 1,0 bar bis 1,5 bar unter Erhalt einer Zusammensetzung Z5 und eines ersten Anteils A1 des Lösungsmittels ausgebildet. In der ersten Kolonne 46 wird die Zusammensetzung Z4 bei einem Druck von 1,0 bar bis 1,5 bar, wie beispielsweise 1,35 bar, teilweise entgast. Dabei wird die Zusammensetzung Z5, der erste Anteil A1 des Lösungsmittels und eine Zusammensetzung Z10 erhalten. Die so entstehende Zusammensetzung Z10, die im Wesentlichen Acetylen und Vinylacetylen enthält, wird dem ersten Stripper 36 als Strippgas über die Leitung 52 wieder zugeführt. Das Strippgas weist in der Leitung 52 einen Druck von 1,30 bar bis 1,40 bar und eine Temperatur von 110 °C bis 120 °C auf. Beispielsweise weist das Strippgas in der Leitung 52 einen Druck von 1,35 bar und eine Temperatur von 115 °C auf. Ein Sumpf in der ersten Kolonne 46 wird über einen Verdampfer 90 erhitzt. Der Verdampfer 90 wird so betrieben, dass der Sumpf bei Wiedereintritt in die erste Kolonne 46 einen Druck von 1,2 bar bis 1,5 bar und eine Temperatur von 165 °C bis 180 °C aufweist. Beispielsweise weist der Sumpf bei Wiedereintritt in die erste Kolonne 46 einen Druck von 1,4 bar und eine Temperatur von 173 °C auf. Das so entstehende Gas, das im Wesentlichen Acetylen und Vinylacetylen enthält, wird dem ersten Stripper 36 als Strippgas über die Leitung 52 wieder zugeführt. Das Strippgas weist in der Leitung 52 einen Druck von 1,30 bar bis 1,40 bar und eine Temperatur von 110 °C bis 120 °C auf. Beispielsweise weist das Strippgas in der Leitung 52 einen Druck von 1,35 bar und eine Temperatur von 115 °C auf. Das Strippgas wird dabei in der Leitung 52 von einem Wärmetauscher 92 gekühlt. Nach dem Wärmetauscher 92 weist das Strippgas einen Druck von 1,0 bar bis 1,50 bar und eine Temperatur von 35 °C bis 45 °C auf. Beispielsweise weist das Strippgas nach dem Wärmetauscher 92 einen Druck von 1,33 bar und eine Temperatur von 40 °C auf. Das aus dem Wärmetauscher 92 entnehmbare Mischkondensat weist in diesem Fall ebenfalls eine Temperatur von 40 °C auf.

Die Zusammensetzung Z5 umfasst Vinylacetylen, Methylacetylen, überschüssiges Prozesswasser und das übrige Lösungsmittel. Die Zusammensetzung Z5 wird als gasförmiger Seitenstrom aus der ersten Kolonne 46 abgezogen. Der Wassergehalt des Lösungsmittels wird kontrolliert durch die Verdampferleistung in der ersten Kolonne 46. Im Sumpf der ersten Kolonne 46 ist die Entgasung abgeschlossen und der erste Anteil A1 des Lösungsmittels wird über eine Leitung 94, über die die erste Kolonne 46 mit dem zweiten Wäscher 26 verbunden ist, wieder dem zweiten Wäscher 26 zugeführt. Alternativ oder zusätzlich kann der erste Anteil A1 des Lösungsmittels über eine nicht näher gezeigte Leitung dem ersten Wäscher 22 zugeführt werden.

Die Zusammensetzung Z5 wird dem zweiten Stripper 56 über die Leitung 54 zugeführt. Die erste Kolonne 46 wird in der oben beschriebenen Weise betrieben. Dadurch weist die die erste Kolonne 46 an dem Seitenabzug 58 verlassende Zusammensetzung Z5 einen Druck von 1,0 bar bis 1,5 bar und eine Temperatur von 140 °C bis 150 °C auf. Beispielsweise weist die die erste Kolonne 46 an dem Seitenabzug 58 verlassende Zusammensetzung Z5 einen Druck von 1,375 bar und eine Temperatur von 147 °C auf. In dem zweiten Stripper 56 wird einer Zusammensetzung Z9, die nachstehend noch näher beschrieben wird, die Zusammensetzung Z5 unter Erhalt eines zweiten Anteils A2 des Lösungsmittels und einer Zusammensetzung Z6 zugeführt. Der zweite Anteil A2 des Lösungsmittels kann über eine Leitung 96, über die der zweite Stripper 56 mit dem zweiten Wäscher 26 verbunden ist, wieder dem zweiten Wäscher26 zugeführt werden. Alternativ oder zusätzlich kann der zweite Anteil A2 des Lösungsmittels über eine nicht näher gezeigte Leitung dem ersten Wäscher 22 zugeführt werden. Der dritte Stripper 68 ist zum Strippen des Lösungsmittels aus dem ersten Wäscher 22 unter Erhalt der Zusammensetzung Z9 ausgebildet. In dem dritten Stripper 68 wird die kleine Menge an Lösungsmittel aus dem ersten Wäscher 22 mit Rohsynthesegas gestrippt, um gelöstes Acetylen auszutreiben und den Brüdenstrom über eine Leitung 98 zur Saugseite des Kompressors 18 zurückzuführen. Dabei wird die Zusammensetzung Z9 erhalten. Der dritte Stripper 68 ist über eine Leitung 100 mit dem zweiten Stripper 56 verbunden. Die Zusammensetzung Z9 wird dem zweiten Stripper 56 über die Leitung 100 an seinem Kopfende 62 zugeführt. Der dritte Stripper 68 wird dabei so betrieben, dass die Zusammensetzung Z9 in der Leitung 100 einen Druck von 1,0 bar bis 1,3 bar und eine Temperatur von 40 °C bis 55 ° aufweist. Beispielsweise weist die Zusammensetzung Z9 in der Leitung 100 einen Druck von 1,22 bar und eine Temperatur von 47,5 °C auf. In dem zweiten Stripper 56 strömt der gasförmige Entnahmestrom der Zusammensetzung Z5 aus der ersten Kolonne 46 im Gegenstrom zum Lösungsmittel der Zusammensetzung Z9. An dieser Stelle sei erwähnt, dass auch der in dem ersten Stripper 36 gebildete Brüdenstrom über die Leitung 98 zur Saugseite des Kompressors 18 zurückgeführt werden kann.

Die in der Nähe des Seitenabzugs 58 der ersten Kolonne 46 angeordnete Vorrichtung 72 zum Versetzen der Zusammensetzung Z5 mit einem Verdünnungsgas versetzt die Zusammensetzung Z5 mit dem Verdünnungsgas, das in die Leitung 54 eingespeist wird. Das Verdünnungsgas ist ausgewählt aus der Gruppe bestehend aus H₂, N₂, CO₂, NH₃, Armgas und Erdgas. Beispielsweise wird Erdgas als Verdünnungsgas und mit einem den Sicherheitsanforderungen entsprechenden Volumenstrom zugeführt. Der zweite Stripper 56 ist zum Entgasen der Zusammensetzung Z5 bei einem Druck von 1,0 bar bis 1,4 bar ausgebildet, beispielsweise 1,22 bar. Bevorzugt ist der Druck in dem zweiten Stripper 56 etwas geringer als in der ersten Kolonne 46, was über die Einspeisungsmenge des Verdünnungsgases gesteuert werden kann. Ein Sumpf in dem zweiten Stripper 56 wird über einen Verdampfer 102 erhitzt. Der Verdampfer 102 wird so betrieben, dass der Sumpf bei Wiedereintritt in den zweiten Stripper 56 einen Druck von 1,20 bar bis 1,30 bar und eine Temperatur von 155 °C bis 170 °C aufweist. Beispielsweise weist der Sumpf bei Wiedereintritt in den zweiten Stripper 56 einen Druck von 1,25 bar und eine Temperatur von 163 °C auf. Dies gilt auch für den zweiten Anteil A2 des Lösungsmittels in der Leitung 96. Entsprechend dieser Vorgehensweise wird die Zusammensetzung Z6 erhalten. Die Zusammensetzung Z6, die den Brüden des zweiten Strippers 56 bildet, umfasst Vinylacetylen, Methylacetylen, überschüssiges Prozesswasser und das übrige Lösungsmittel.

Die Zusammensetzung Z6 wird der zweiten Kolonne 76 über die Leitung 74 zugeführt. Die Zusammensetzung Z6 weist durch die oben beschriebene Betriebsweise des zweiten Strippers 56 in der Leitung 74 einen Druck von 1,0 bar bis 1,4 bar und eine Temperatur von 120 °C bis 130 °C auf. Beispielsweise weist die Zusammensetzung Z6 in der Leitung 74 einen Druck von 1,22 bar und eine Temperatur von 125 °C auf. Die zweite Kolonne 76 ist zum Versetzen der Zusammensetzung Z6 mit Wasser unter Erhalt einer Zusammensetzung Z7 enthaltend einen dritten Anteil A3 des Lösungsmittels und Wasser sowie unter Erhalt einer Zusammensetzung Z8 enthaltend das substituierte Acetylen ausgebildet. Mit anderen Worten wird das noch in der Zusammensetzung Z6 enthaltene oder verbliebene Lösungsmittel mit wenig Wasser zurückgewaschen. Das der zweiten Kolonne 76 zugeführte Wasser kann mit einem Massenstrom von 2000 kg/h bis 2400 kg/h zugeführt werden, beispielsweise 2200 kg/h. Das so gebildete Wasser-Lösungsmittel-Gemisch kann dem Hauptlösungsmittelstrom in der Leitung 44 wieder zugeführt werden. Die zweite Kolonne 76 wird so betrieben, dass das Wasser-Lösungsmittel-Gemisch an dem unteren Ende 78 entnehmbar ist und eine Temperatur von 100 °C bis 110 ° aufweist, beispielsweise 104 °C.

Die Zusammensetzung Z8 wird dem Mischkondensator 82 über die Leitung 80 zugeführt. Die zweite Kolonne 76 wird so betrieben, dass die Zusammensetzung Z8 in der Leitung 80 einen Druck von 1,0 bar bis 1,3 bar und eine Temperatur von 90 °C bis 110 ° C aufweist. Beispielsweise weist die Zusammensetzung Z8 in der Leitung 80 einen Druck von 1,18 bar und eine Temperatur von 100 °C auf. Der Mischkondensator 82 ist zum Versetzen der Zusammensetzung Z8 mit Wasser unter Erhalt einer Zusammensetzung Z11 enthaltend das substituierte Acetylen ausgebildet. In dem Mischkondensator 82 wird die Zusammensetzung Z8 mit Wasser unter Erhalt der Zusammensetzung Z11 enthaltend das substituierte Acetylen versetzt. Das dem Mischkondensator 82 zugeführte Wasser weist eine Temperatur von 40 °C bis 60 °C auf, beispielsweise 50 °C. Die gasförmige Zusammensetzung Z8 wird dabei durch Direktkontakt mit Wasser in dem Mischkondensator 82 gekühlt, um den Großteil des Wasserdampfes auszukondensieren. Das an dem unteren Ende 86 entnehmbare Wasser weist in diesem Fall eine Temperatur von etwa 60 °C auf, insbesondere zwischen 40 °C und 80 °C und vorzugsweise 60 °C bis 70 °C, wird über einen Wärmetauscher 104 gekühlt und wieder dem Mischkondensator 82 mit der oben beschriebenen Temperatur zugeführt. Optional kann Wasser in diesen Kreislauf eingespeist oder aus diesem Kreislauf entnommen werden. Das substituierte Acetylen wird an dem Kopfende 88 des Mischkondensators 82 entnommen. Das an dem Kopfende 88 des Mischkondensator 82 entnommene substituierte Acetylen weist bei der beschriebenen Betriebsweise einen Druck von 1,0 bar bis 1,3 bar und eine Temperatur von 45 °C bis 65 °C auf. Beispielsweise weist das an dem Kopfende 88 des Mischkondensator 82 entnommene substituierte Acetylen einen Druck von 1,15 bar und eine Temperatur von 50 °C bis 60 °C auf.

Durch die beschriebene Betriebsweise der Vorrichtung 10 bei Normaldruck werden die herkömmliche Vakuummaschine zum Verdichten der Zusammensetzung Z10 und die Gasmaschine und Kühlvorrichtung zum Verdichten der Zusammensetzung Z11 überflüssig. Stattdessen ergibt sich eine sichere und kostengünstigere Betriebsweise bei Normaldruck bzw. geringem Überdruck. Dadurch verlagert sich die kritische Stelle für Naphthalin in den Mischkondensator 82. Die Temperatur in dem Mischkondensator 82 wird daher so eingestellt, dass ein Ausfall von Naphthalin verhindert wird. Die Sumpftemperatur wird beispielsweise auf 60°C bis 70 °C eingestellt, wodurch der Ausfall von Naphthalin vermieden wird. Stromabwärts des Mischkondensators 82 kann durch Verdünnen mit beispielsweise Erdgas oder Armgas der Abstand vom Taupunkt vergrößert werden, so dass Kondensation auf dem Weg zur Fackel vermieden werden kann.

### Bezugszeichenliste

- 10: Vorrichtung
- 12: Reaktor
- 14: Brennerblock
- 16: Leitung
- 18: Kompressor
- 20: Leitung
- 22: Erster Wäscher
- 24: Leitung
- 26: Zweiter Wäscher
- 28: Kopfende
- 30: Unteres seitliches Ende
- 32: Kopfende
- 34: Leitung
- 36: Erster Stripper
- 38: Unteres Ende
- 40: Kopfende
- 42: Seite
- 44: Leitung
- 46: Erste Kolonne
- 48: Unteres Ende
- 50: Kopfende
- 52: Leitung
- 54: Leitung
- 56: Zweiter Stripper
- 58: Seitenabzug
- 60: Unteres Ende
- 62: Kopfende
- 64: Unteres Ende
- 66: Leitung
- 68: Dritter Stripper
- 70: Kopfende
- 72: Vorrichtung zum Zuführen eines Verdünnungsgases
- 74: Leitung
- 76: Zweite Kolonne
- 78: Unteres Ende
- 80: Leitung
- 82: Mischkondensator
- 84: Kopfende
- 86: Unteres Ende
- 88: Kopfende
- 90: Verdampfer
- 92: Wärmetauscher
- 94: Leitung
- 96: Leitung
- 98: Leitung
- 100: Leitung
- 102: Verdampfer
- 104: Wärmetauscher

## Patentansprüche

1. Vorrichtung (10) zum Herstellen von Acetylen und Synthesegas durch partielle Oxidation von Kohlenwasserstoffen mit Sauerstoff, umfassend
einen Reaktor (12), wobei der Reaktor (12) einen Brennerblock (14) mit einem Feuerraum zur Herstellung einer Zusammensetzung Z1 enthaltend zumindest Acetylen und substituiertes Acetylen aufweist,
einen ersten Wäscher (22), der zum Versetzen der Zusammensetzung Z1 mit einem Lösungsmittel unter Erhalt einer Zusammensetzung Z2 ausgebildet ist,
einen zweiten Wäscher (26), der zum Versetzen der Zusammensetzung Z2 mit dem Lösungsmittel unter Erhalt einer Zusammensetzung Z3 ausgebildet ist,
einen ersten Stripper (36), der zum Strippen der Zusammensetzung Z3 unter Erhalt einer Zusammensetzung Z4 enthaltend das substituierte Acetylen, Acetylen und das Lösungsmittel und zum Abtrennen des Acetylens ausgebildet ist,
eine erste Kolonne (46), die zum teilweisen Entgasen der Zusammensetzung Z4 bei einem Druck von 1,0 bar bis 1,5 bar unter Erhalt einer Zusammensetzung Z5, einer Zusammensetzung Z10 und eines ersten Anteils A1 des Lösungsmittels ausgebildet ist,
einen zweiten Stripper (56), dem zum Strippen einer Zusammensetzung Z9 die Zusammensetzung Z5 unter Erhalt eines zweiten Anteils A2 des Lösungsmittels und einer Zusammensetzung Z6 zuführbar ist,
einen dritten Stripper (68), der zum Strippen des Lösungsmittels aus dem ersten Wäscher (22) unter Erhalt einer Zusammensetzung Z9 ausgebildet ist, wobei der dritte Stripper (68) zum Zuführen der Zusammensetzung Z9 zu dem zweiten Stripper (56) mit dem zweiten Stripper (56) verbunden ist,
wobei zwischen der ersten Kolonne (46) und dem zweiten Stripper (56) eine Vorrichtung (72) zum Versetzen der Zusammensetzung Z5 mit einem Verdünnungsgas angeordnet ist,
eine zweite Kolonne (76), die zum Versetzen der Zusammensetzung Z6 mit Wasser unter Erhalt einer Zusammensetzung Z7 enthaltend einen dritten Anteil A3 des Lösungsmittels und Wasser und unter Erhalt einer Zusammensetzung Z8 enthaltend das substituierte Acetylen ausgebildet ist, und
einen Mischkondensator (82), der zum Versetzen der Zusammensetzung Z8 mit Wasser unter Erhalt einer Zusammensetzung Z11 enthaltend das substituierte Acetylen ausgebildet ist.

2. Vorrichtung (10) nach dem vorhergehenden Anspruch, wobei das Verdünnungsgas ausgewählt ist aus der Gruppe bestehend aus: H₂, N₂, CO₂, NH₃, Armgas, Erdgas.

3. Vorrichtung (10) nach einem der vorhergehenden Ansprüche, wobei der aus dem zweiten Stripper (56) erhaltene zweite Anteil A2 des Lösungsmittels dem ersten und/oder zweiten Wäscher (22, 26) zuführbar ist.

4. Vorrichtung (10) nach einem der vorhergehenden Ansprüche, wobei der aus der ersten Kolonne (46) erhaltene erste Anteil A1 des Lösungsmittels dem ersten und/oder zweiten Wäscher (22, 26) zuführbar ist.

5. Vorrichtung (10) nach einem der vorhergehenden Ansprüche, wobei die erste Kolonne (46) zum Abführen der Zusammensetzung Z5 einen Seitenabzug (58) aufweist, wobei die Vorrichtung (72) zum Versetzen der Zusammensetzung Z5 mit einem Verdünnungsgas an den Seitenabzug (58) angrenzt.

6. Vorrichtung (10) nach einem der vorhergehenden Ansprüche, wobei eine Temperatur in dem Mischkondensator (82) zum Verhindern eines Ausfalls von Naphthalin eingestellt ist.

7. Vorrichtung (10) nach dem vorhergehenden Anspruch, wobei die Temperatur in dem Mischkondensator (82) von 40 °C bis 80 °C ist.

8. Vorrichtung (10) nach einem der vorhergehenden Ansprüche, wobei der zweite Stripper (56) zum Entgasen der Zusammensetzung Z9 bei einem Druck von 1,0 bar bis 1,4 bar betrieben wird.

9. Vorrichtung (10) nach einem der vorhergehenden Ansprüche, wobei das Lösungsmittel N-Methyl-2-pyrrolidon ist.

10. Verfahren zum Herstellen von Acetylen und Synthesegas durch partielle Oxidation von Kohlenwasserstoffen mit Sauerstoff, umfassend
partielles Oxidieren von Kohlenwasserstoffen mit Sauerstoff zum Herstellen einer Zusammensetzung Z1 enthaltend zumindest Acetylen und substituiertes Acetylen, Versetzen der Zusammensetzung Z1 mit einem Lösungsmittel unter Erhalt einer Zusammensetzung Z2,
Versetzen der Zusammensetzung Z2 mit dem Lösungsmittel unter Erhalt einer Zusammensetzung Z3,
Strippen der Zusammensetzung Z3 unter Erhalt einer Zusammensetzung Z4 enthaltend das substituierte Acetylen, Acetylen und das Lösungsmittel und zum Abtrennen des Acetylens aus der Zusammensetzung Z3,
teilweises Entgasen der Zusammensetzung Z4 bei einem Druck von 1,0 bar bis 1,5 bar unter Erhalt einer Zusammensetzung Z5, einer Zusammensetzung Z10 und eines ersten Anteils A1 des Lösungsmittels,
Strippen einer Zusammensetzung Z9 mit der Zusammensetzung Z5 unter Erhalt eines zweiten Anteils A2 des Lösungsmittels und einer Zusammensetzung Z6,
Strippen des nach der Herstellung der Zusammensetzung Z2 erhaltenen Lösungsmittels unter Erhalt der Zusammensetzung Z9,
Versetzen der Zusammensetzung Z5 mit einem Verdünnungsgas,
Versetzen der Zusammensetzung Z6 mit Wasser unter Erhalt einer Zusammensetzung Z7 enthaltend einen dritten Anteil A3 des Lösungsmittels und Wasser und unter Erhalt einer Zusammensetzung Z8 enthaltend das substituierte Acetylen, und
Versetzen der Zusammensetzung Z8 mit Wasser unter Erhalt einer Zusammensetzung Z11 enthaltend das substituierte Acetylen.

11. Verfahren nach dem vorhergehenden Anspruch, wobei das Verdünnungsgas ausgewählt ist aus der Gruppe bestehend aus: H₂, N₂, CO₂, NH₃, Armgas, Erdgas.

12. Verfahren nach einem der beiden vorhergehenden Ansprüche, wobei der erhaltene zweite Anteil A2 des Lösungsmittels der Zusammensetzung Z1 und/oder Z2 zugeführt wird.

13. Verfahren nach einem der drei vorhergehenden Ansprüche, wobei der erhaltene erste Anteil A1 des Lösungsmittels der Zusammensetzung Z1 und/oder Z2 zugeführt wird.

14. Verfahren nach einem der vier vorhergehenden Ansprüche, wobei die Zusammensetzung Z8 in einem Mischkondensator (82) mit dem Wasser versetzt wird, wobei eine Temperatur in dem Mischkondensator zum Verhindern eines Ausfalls von Naphthalin eingestellt wird,

15. Verfahren nach dem vorhergehenden Anspruch, wobei die Temperatur von 40 °C bis 80 °C ist.

16. Verfahren nach einem der sechs vorhergehenden Ansprüche, wobei die Zusammensetzung Z9 bei einem Druck von 1,0 bar bis 1,4 bar entgast wird.

17. Verfahren nach einem der sieben vorhergehenden Ansprüche, wobei das Lösungsmittel N-Methyl-2-pyrrolidon ist.

## Claims

1. An apparatus (10) for the preparation of acetylene and synthesis gas by partial oxidation of hydrocarbons with oxygen, comprising
a reactor (12), wherein the reactor (12) comprises a burner block (14) with a furnace chamber for the preparation of a composition C1 comprising at least acetylene and substituted acetylene,
a first scrubber (22) which is constructed for adding a solvent to the composition C1 to obtain a composition C2,
a second scrubber (26) which is constructed for adding a solvent to the composition C2 to obtain a composition C3,
a first stripper (36) which is constructed for stripping the composition C3 to obtain a composition C4 comprising the substituted acetylene, acetylene and the solvent and for separating off the acetylene, a first column (46) which is constructed for partial degassing of the composition C4 under a pressure of from 1.0 bar to 1.5 bar to obtain a composition C5, a composition C10 and a first amount A1 of the solvent,
a second stripper (56) to which the composition C5 can be fed for stripping a composition C9 to obtain a second amount A2 of the solvent and a composition C6,
a third stripper (68) which is constructed for stripping the solvent from the first scrubber (22) to obtain a composition C9, the third stripper (68) being connected to the second stripper (56) for feeding the composition C9 to the second stripper (56),
wherein an apparatus (72) for adding a diluting gas to the composition C5 is arranged between the first column (46) and the second stripper (56),
a second column (76) which is constructed for adding water to the composition C6 to obtain a composition C7 comprising a third amount A3 of the solvent and water and to obtain a composition C8 comprising the substituted acetylene, and
a mixing condenser (82) which is constructed for adding water to the composition C8 to obtain a composition C11 comprising the substituted acetylene.

2. The apparatus (10) as claimed in the preceding claim, wherein the diluting gas is selected from the group consisting of: H₂, N₂, CO₂, NH₃, lean gas, natural gas.

3. The apparatus (10) as claimed in one of the preceding claims, wherein the second amount A2 of the solvent obtained from the second stripper (56) can be fed to the first and/or second scrubber (22, 26).

4. The apparatus (10) as claimed in one of the preceding claims, wherein the first content A1 of the solvent obtained from the first column (46) can be fed to the first and/or second scrubber (22, 26).

5. The apparatus (10) as claimed in one of the preceding claims, wherein the first column (46) has a side take-off (58) for leading off the composition C5, wherein the apparatus (72) for adding a diluting gas to the composition C5 is adjacent to the side take-off (58) .

6. The apparatus (10) as claimed in one of the preceding claims, wherein a temperature is established in the mixing condenser (82) for preventing a precipitation of naphthalene.

7. The apparatus (10) as claimed in the preceding claim, wherein the temperature in the mixing condenser (82) is from 40°C to 80°C.

8. The apparatus (10) as claimed in one of the preceding claims, wherein the second stripper (56) for degassing the composition C9 can be operated under a pressure of from 1.0 bar to 1.4 bar.

9. The apparatus (10) as claimed in one of the preceding claims, wherein the solvent is N-methyl-2-pyrrolidone.

10. A process for the preparation of acetylene and synthesis gas by partial oxidation of hydrocarbons with oxygen, comprising
partial oxidation of hydrocarbons with oxygen for the preparation of a composition C1 comprising at least acetylene and substituted acetylene,
addition of a solvent to the composition C1 to obtain a composition C2,
addition of the solvent to the composition C2 to obtain a composition C3,
stripping of the composition C3 to obtain a composition C4 comprising the substituted acetylene, acetylene and the solvent and for separating off the acetylene from the composition C3,
partial degassing of the composition C4 under a pressure of from 1.0 bar to 1.5 bar to obtain a composition C5, a composition C10 and a first amount A1 of the solvent,
stripping of a composition C9 with the composition C5 to obtain a second amount A2 of the solvent and a composition C6,
stripping of the solvent obtained after preparation of the composition C2 to obtain the composition C9, addition of a diluting gas to the composition C5, addition of water to the composition C6 to obtain a composition C7 comprising a third amount A3 of the solvent and water and to obtain a composition C8 comprising the substituted acetylene, and
addition of water to the composition C8 to obtain a composition C11 comprising the substituted acetylene.

11. The process as claimed in the preceding claim, wherein the diluting gas is selected from the group consisting of: H₂, N₂, CO₂, NH₃, lean gas, natural gas.

12. The process as claimed in one of the two preceding claims, wherein the second amount A2 of the solvent obtained is fed to the composition C1 and/or C2.

13. The process as claimed in one of the three preceding claims, wherein the first amount A1 of the solvent obtained is fed to the composition C1 and/or C2.

14. The process as claimed in one of the four preceding claims, wherein the water is added to the composition C8 in a mixing condenser (82), wherein a temperature is established in the mixing condenser for preventing a precipitation of naphthalene.

15. The process as claimed in the preceding claim, wherein the temperature is from 40°C to 80°C.

16. The process as claimed in one of the six preceding claims, wherein the composition C9 is degassed under a pressure of from 1.0 bar to 1.4 bar.

17. The process as claimed in one of the seven preceding claims, wherein the solvent is N-methyl-2-pyrrolidone.

## Revendications

1. Dispositif (10) pour la fabrication d'acétylène et d'un gaz de synthèse par oxydation partielle d'hydrocarbures avec de l'oxygène, comprenant :
un réacteur (12), le réacteur (12) comprenant un bloc brûleur (14) comprenant une chambre de combustion pour la fabrication d'une composition Z1 contenant au moins de l'acétylène et de l'acétylène substitué,
un premier épurateur (22), qui est conçu pour le mélange de la composition Z1 avec un solvant pour obtenir une composition Z2,
un deuxième épurateur (26), qui est conçu pour le mélange de la composition Z2 avec le solvant pour obtenir une composition Z3,
un premier extracteur (36), qui est conçu pour l'extraction de la composition Z3 pour obtenir une composition Z4 contenant l'acétylène substitué,
l'acétylène et le solvant et pour la séparation de l'acétylène,
une première colonne (46), qui est conçue pour le dégazage partiel de la composition Z4 à une pression de 1,0 bar à 1,5 bar pour obtenir une composition Z5, une composition Z10 et une première fraction A1 du solvant,
un deuxième extracteur (56), dans lequel la composition Z5 peut être introduite pour l'extraction d'une composition Z9 pour obtenir une deuxième fraction A2 du solvant et une composition Z6,
un troisième extracteur (68), qui est conçu pour l'extraction du solvant du premier épurateur (22) pour obtenir une composition Z9, le troisième extracteur (68) étant raccordé avec le deuxième extracteur (56) pour l'introduction de la composition Z9 dans le deuxième extracteur (56),
un dispositif (72) pour le mélange de la composition Z5 avec un gaz diluant étant agencé entre la première colonne (46) et le deuxième extracteur (56),
une deuxième colonne (76), qui est conçue pour le mélange de la composition Z6 avec de l'eau pour obtenir une composition Z7 contenant une troisième fraction A3 du solvant et de l'eau et pour obtenir une composition Z8 contenant l'acétylène substitué, et
un condensateur de mélange (82) qui est conçu pour le mélange de la composition Z8 avec de l'eau pour obtenir une composition Z11 contenant l'acétylène substitué.

2. Dispositif (10) selon la revendication précédente, dans lequel le gaz diluant est choisi dans le groupe constitué par : H₂, N₂, CO₂, NH₃, un gaz pauvre, le gaz naturel.

3. Dispositif (10) selon l'une quelconque des revendications précédentes, dans lequel la deuxième fraction A2 du solvant obtenue à partir du deuxième extracteur (56) peut être introduite dans le premier et/ou le deuxième épurateur (22, 26).

4. Dispositif (10) selon l'une quelconque des revendications précédentes, dans lequel la première fraction A1 du solvant obtenue à partir de la première colonne (46) peut être introduite dans le premier et/ou le deuxième épurateur (22, 26).

5. Dispositif (10) selon l'une quelconque des revendications précédentes, dans lequel la première colonne (46) comprend un soutirage latéral (58) pour le déchargement de la composition Z5, le dispositif (72) pour le mélange de la composition Z5 avec un gaz diluant étant adjacent au soutirage latéral (58).

6. Dispositif (10) selon l'une quelconque des revendications précédentes, dans lequel une température dans le condensateur de mélange (82) est ajustée pour empêcher une précipitation de naphtaline.

7. Dispositif (10) selon la revendication précédente, dans lequel la température dans le condensateur de mélange (82) est de 40 °C à 80 °C.

8. Dispositif (10) selon l'une quelconque des revendications précédentes, dans lequel le deuxième extracteur (56) pour le dégazage de la composition Z9 est exploité à une pression de 1,0 bar à 1,4 bar.

9. Dispositif (10) selon l'une quelconque des revendications précédentes, dans lequel le solvant est la N-méthyl-2-pyrrolidone.

10. Procédé de fabrication d'acétylène et d'un gaz de synthèse par oxydation partielle d'hydrocarbures avec de l'oxygène, comprenant :
l'oxydation partielle d'hydrocarbures avec de l'oxygène pour la fabrication d'une composition Z1 contenant au moins de l'acétylène et de l'acétylène substitué,
le mélange de la composition Z1 avec un solvant pour obtenir une composition Z2,
le mélange de la composition Z2 avec le solvant pour obtenir une composition Z3,
l'extraction de la composition Z3 pour obtenir une composition Z4 contenant l'acétylène substitué, l'acétylène et le solvant, et pour séparer l'acétylène de la composition Z3,
le dégazage partiel de la composition Z4 à une pression de 1,0 bar à 1,5 bar pour obtenir une composition Z5, une composition Z10 et une première fraction A1 du solvant, l'extraction d'une composition Z9 avec la composition Z5 pour obtenir une deuxième fraction A2 du solvant et une composition Z6,
l'extraction du solvant obtenu après la fabrication de la composition Z2 pour obtenir la composition Z9,
le mélange de la composition Z5 avec un gaz diluant,
le mélange de la composition Z6 avec de l'eau pour obtenir une composition Z7 contenant une troisième fraction A3 du solvant et de l'eau et pour obtenir une composition Z8 contenant l'acétylène substitué, et
le mélange de la composition Z8 avec de l'eau pour obtenir une composition Z11 contenant l'acétylène substitué.

11. Procédé selon la revendication précédente, dans lequel le gaz diluant est choisi dans le groupe constitué par : H₂, N₂, CO₂, NH₃, un gaz pauvre, le gaz naturel.

12. Procédé selon l'une quelconque des deux revendications précédentes, dans lequel la deuxième fraction A2 du solvant obtenue est introduite dans la composition Z1 et/ou Z2.

13. Procédé selon l'une quelconque des trois revendications précédentes, dans lequel la première fraction A1 du solvant obtenue est introduite dans la composition Z1 et/ou Z2.

14. Procédé selon l'une quelconque des quatre revendications précédentes, dans lequel la composition Z8 est mélangée avec de l'eau dans un condensateur de mélange (82), une température dans le condensateur de mélange étant ajustée pour empêcher une précipitation de naphtaline.

15. Procédé selon la revendication précédente, dans lequel la température est de 40 °C à 80 °C.

16. Procédé selon l'une quelconque des six revendications précédentes, dans lequel la composition Z9 est dégazée à une pression de 1,0 bar à 1,4 bar.

17. Procédé selon l'une quelconque des sept revendications précédentes, dans lequel le solvant est la N-méthyl-2-pyrrolidone.
